# EUROPEAN PATENT APPLICATION

(11) **EP 1 992 339 A1**
(43) Date of publication of application: **19.11.2008**
(21) Application number: 07009881.9
(22) Date of filing: 18.05.2007
(51) Int. Cl.: A61K 31/01, A61K 31/409, A61K 31/555, A61K 33/06, A61K 47/02, A61K 47/48, A61K 49/06, A61K 9/00

(54) **Use of a magnesium isotope for treating hypoxia and a medicament comprising the same**

(71) Applicant: Sarkar, Saeed, Abad-Tehran (IR); Rezayat,, Seyed Mahdi, Tehran (IR); Buchachenko, Anatoly Leonidovich, Moscow 117420 (RU); Kuznetsov, Dmitry Anatolevich, Moscow 117342 (RU); Orlova, Marina Alexeyevna, Moscow 117342 (RU); Yurovskaya, Marina Abramovna, Moscow 117449 (RU); Trushkov, Igor Viktorovich, Moscow 109542 (RU)
(72) Inventor: Sarkar, Saeed, Abad-Tehran (IR); Rezayat,, Seyed Mahdi, Tehran (IR); Buchachenko, Anatoly Leonidovich, Moscow 117420 (RU); Kuznetsov, Dmitry Anatolevich, Moscow 117342 (RU); Orlova, Marina Alexeyevna, Moscow 117342 (RU); Yurovskaya, Marina Abramovna, Moscow 117449 (RU); Trushkov, Igor Viktorovich, Moscow 109542 (RU)
(74) Representative: Fuchs

(57) **Abstract**

The present invention describes the use of the magnetic magnesium isotope ²⁵Mg²⁺ for the manufacture of a medicament for treating hypoxia. Furthermore, the present invention provides a medicament for treating hypoxia comprising the magnetic magnesium isotope ²⁵Mg²⁺ and a porphylleren compound according to formula (1) 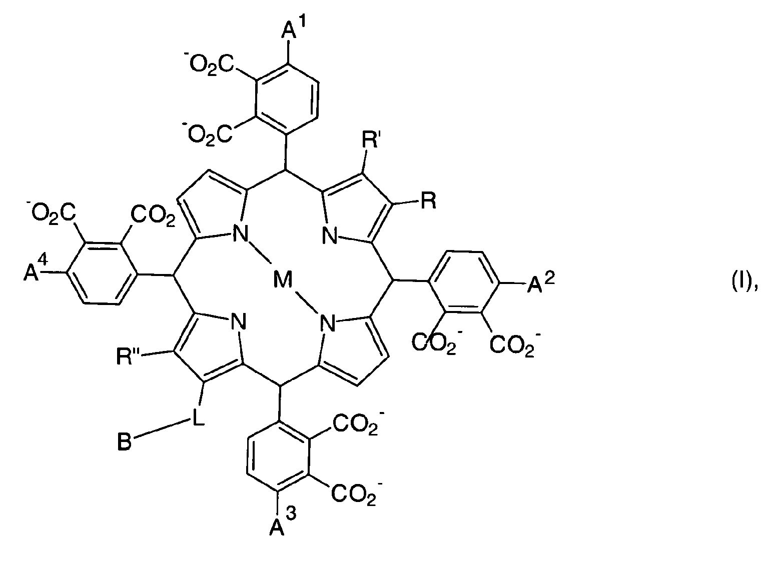 wherein the residues A¹, A², A³, and A⁴ independently are a group containing 1 to 40 carbon atoms and at least one carboxylic group, the residues R, R' and R" independently are hydrogen or a group containing 1 to 80 carbon atoms, M is 2 H or an element capable of complexing with pyrrole nitrogen atom and L is a linking group and B is a fullerene residue.

## Description

The present invention relates to the use of a ²⁵Mg²⁺ for the manufacture of a medicament for treating hypoxia and the same medicament.

All hypoxia-relieving therapies ever tested (ATP, Panangin (Russia), O₂ inhalation, Magnesium salts, etc) are not "smart" ones, i.e. their action *in vivo* is uncontrollable by the body itself, i.e. by means of homeostasis. In other words, these therapies are only useful when the signs of hypoxia are already observed in the patient and the drugs can only be applied after the problem has come up.

In the case of therapies involving the inhalation of oxygen, the fact that the required equipment is not available when needed and the difficulty and/or danger of dealing with oxygen cylinders should also be considered as a significant drawback.

Another drawback of existing hypoxia therapies is the lack of selectivity regarding the mechanism of treatment, that is, the target tissue for which the treatment specifically works cannot be specified.

Hence, there exists a strong need for a new way of treating hypoxia and a respective medicament, which medicament can supply a compound acting as Active Pharmaceutical Ingredient (API) for treating hypoxia. The supply or release should be smart and open the way for effective and easy-to-use application in the treatment of hypoxia for different tissues. According to preferred embodiments, the heart tissue is the target tissue.

The use of the API and the respective medicament achieving its smart delivery should further also open the possibility for treatment of hypoxia in different parts of the body, even with selective delivery of the API to the particular targets.

Furthermore, the drug or medicament should have the ability to accumulate in the desired target tissues for a specific period, and also the ability to smartly release after the onset of hypoxia. In other words, it can also be used to prevent severe hypoxia and/or prevent or at least postpone the dangerous consequences of hypoxia at least until further measures can be taken.

Summing up, it is an object of the present invention to solve the problems mentioned before.

In particular, it is an object of the present invention to provide a medicament having low toxicity and a good efficiency to treat hypoxia with tissue selectivity if needed.

These objects are solved by the use of the magnetic magnesium isotope ²⁵Mg²⁺ for the manufacture of a medicament for treating hypoxia.

Furthermore, the present invention provides a medicament for treating hypoxia comprising the magnetic magnesium isotope ²⁵Mg²⁺ and a porphylleren compound according to formula (1) 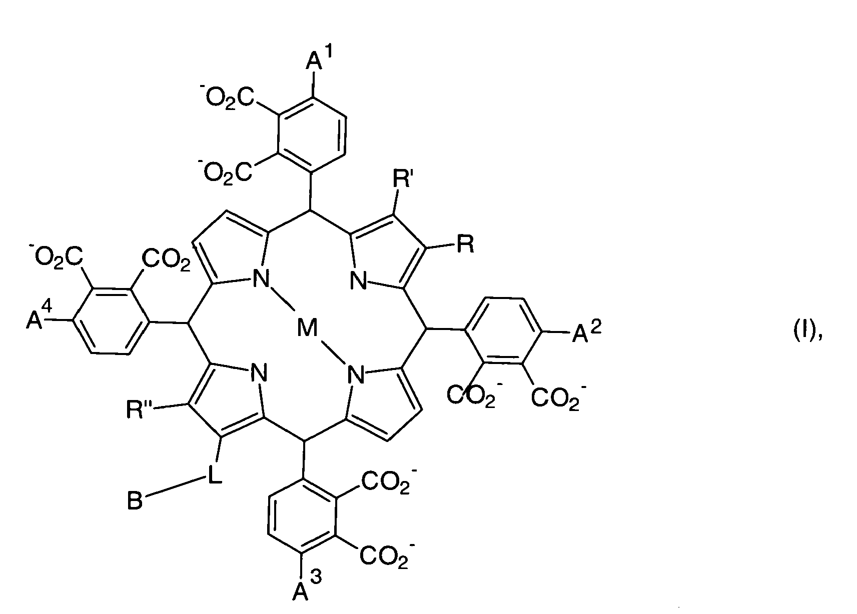 where the residues A¹, A², A³, and A⁴ independently are a group containing 1 to 40 carbon atoms and at least one carboxylic group, the residues R, R' and R" independently are hydrogen or a group containing 1 to 80 carbon atoms, M is 2 H or an element capable of complexing with pyrrole nitrogen atom and L is a linking group and B is a fullerene residue.

A nuclear spin selectivity of the ²⁵Mg²⁺- dependent mitochondrial ATP synthesis pathways is found to be a background for the corrective and effective intervention of hypoxia related metabolic disorders, although the present invention is not bound to this theory. The exclusively high capability of ²⁵Mg²⁺, i.e. the only magnetic magnesium isotope, to activate merely all kinase-promoted processes of nucleotides phosphorylation in mitochondria is believed to be a basis for the successful treatment. Surprisingly, the ATP depletion in a cell can be corrected by a targeted delivery of ultra-micro-amounts (pico-grams, i.e. 10 ⁻¹² g) of ²⁵Mg²⁺ isotope.

According to a preferred aspect of the present invention, the medicament ca achieve a targeted and selective delivery of the ²⁵Mg²⁺ to any muscle. Preferably, the medicament corrects the energy metabolism disorders in myocardiocytes.

Moreover, the medicament may increase the efficiency of the treatment of the hypoxia suffering muscles and decreases the required dose of ²⁵Mg²⁺ required for the treatment.

According to a special embodiment of the present invention, the medicament preferably comprises a target-selective ion carrier. The ion carrier may possess a high degree of water solubility and the ability to be dispersed and retained in lipid layer of biological membranes.

Astonishingly, the present invention provides a medicament possessing a selective delivery and smart release of the ²⁵Mg²⁺ isotope. E.g. specific structural, magnetic, and/or chemical properties of the tissue can be used for the selective delivery and smart release. In the sense of this invention the term "smart" means that the drug is able to release the ²⁵Mg²⁺ isotope only in the case there appears hypoxia in the tissue, i.e. only in the scenario of the disease which leads to acidosis and other symptoms. If not necessary (no hypoxia), there will be no release.

According to a preferred embodiment of the invention, the aims of the invention can be fulfilled through the application of target-selective ion carriers preferably possessing both high degrees of water solubility and the ability to be dispersed and retained in lipid layer of biological membranes.

According to another preferred embodiment of the invention, the aims of the invention can be fulfilled by the application of basic salts of the ion carriers with ²⁵Mg²⁺.

According to another preferred embodiment of the invention, the aims of the invention can be fulfilled by the application of specially designed and synthesized derivatives of porphyrin-adducted nanocationite particles.

According to another preferred embodiment of the invention, the derivatives of porphyrin-adducted nanocationite particles may also be used to deliver and smartly release ²⁵Mg²⁺ or any other cationic species of desire to cells.

According to another preferred embodiment of the invention, the ion carriers possess both high degrees of water solubility (460 mg/ml in the case of PMC16) and the ability to be dispersed and retained in lipid layer of biological membranes, due to their chemical structures.

According to another preferred embodiment of the invention the target selectivity of the ion carrier species can be adjusted regarding the specific characteristics of the target tissue, the chemical structure of the carrier, nature of the metal(s) in the metal porphyry, and the magnetic properties of the carrier.

According to another embodiment of the invention, the choice of metal in the metal porphyry can affect the target selectivity or the functioning of the drugs.

According to another preferred embodiment of the invention the aims of the invention can be fulfilled by most preferably the application of a specific derivative of the mentioned family namely PMC16 *(fullerene(C₆₀)-2-(butadiene-1-yl)-tetra(o-y-aminobutyryl-o-phtalyl) metaloporphyrin,* in which the metal in the metal porphyry can be chosen from any desired metal(s), in the periodic table and one specific or a mixture of their isotopes either in the naturally abundant form or in a form enriched with one specific isotope, or a combination thereof. The choice of the metal, as said before, can affect the target selectivity or the functioning of the drug.

According to another preferred embodiment in case the target tissue is to be that of heart, the most preferred ion carrier is PMC 16 *(fullerene(C₆₀)-2-(butadiene-1-yl)-tetra(o-y-aminobutyryl-o-phtalyl)* metaloporphyrin, in which the metal in the metal porphyry is a magnetic isotope of any desired metal ion.

According to another preferred embodiment of the present invention, in the case of the target tissue being be that of heart, the most preferred ion carrier is PMC16 *(fullerene(C₆₀)-2-(butadiene-1-yl)-tetra(o-y-aminobutyryl-o-phtalyl)* ironporphyrin in which the iron ion is most preferably Fe(III).

According to another preferred embodiment of the invention, ²⁵Mg²⁺ may be delivered to any other desired target in the form of a salt with the PMC16 derivative iron complex, by changing the properties of the drug and/or complex.

According to another preferred embodiment of the invention, the tissue-selectivity of the delivery of the mentioned isotope by PMC16, in the case of heart can be increased by means of the application of the *(fullerene (C₆₀)-2- (butadiene-1-yl) -tetra (o-y-aminobutylyl-o-ph*talyl) metaloporphyrin carriers wherein the metal in the metaloporphyrinis any magnetic element that can be selectivily pulled towards the heart muscle.

According to another embodiment of the present invention due to the special characteristics of porphyrin derivatives, especially PMC16 in the form of a complex with ²⁵Mg²⁺, they can accumulate in the target tissue for a certain period of time, and be smartly released in the target tissue only after hypoxia attacks.

According to another embodiment of the invention, the release of ²⁵Mg²⁺ ion in the target happens through an intelligent mechanism, according to which the isotope is only released in the case of hypoxia.

According to another embodiment of the invention, ²⁵Mg²⁺ is only released quantitatively in the target tissue, in the case of the hypoxia and the consequent acidosis and due to the metabolic decay and transformation of the drug and due to the properties of the drug.

According to another feature of the invention, the drug may also prevent the energy losses in cells and tissues in case of the prior-to-hypoxia administration. So long-term courses of the drug may serve to simultaneous treatment and prevention of hypoxias in patience with a chronic heart failure and related pathologies as well as it might be efficient to prepare people for any anticipated (forthcomings) physiological situation, which may lead to hypoxia (e. g. sports, heavy physical work, stress).

According to another feature of the present invention, because the biological membrane functioning requires (ATP, GTP hydrolysis related energy supply), and as long as the cell life span depends on membrane functioning, the proposed drug may provide an additional "guarantee" for a non-stop (regardless on the oxygen accessibility) energy production mechanism, in the harmful energy losses in the cell in the case of hypoxia and hence prolong the life span of a cell.

According to another feature of the present invention, because the excess of bio-oxidation activity in a cell (including all known peroxide-forming reactions) harms the cell membranes depriving them of being involved properly in to pathways, then the product proposed may help to minimize the consignment energy losses due to the over activation of ATP production in ²⁵Mg-²⁺CK reaction.

According to another feature of the present invention, because an increase in lipid peroxidation rate, may harm not only biological membranes but also the membrane-associated ATP-synthase, and NAD(FAD), dehydrogenates cascade as well, which in turn creates a condition for a sharp ATP production, this might be compensated by activation of the CK-dissected ATP synthesis which is supposed to be activated by the presence of ²⁵Mg²⁺.

According to another feature of the present invention, because both tough-UV and f-ionizing radiations are known to induce truly chaos-leading devastating impacts towards the eukaryotic cell energy megalopolis the delivery of ²⁵Mg-²⁺ might be also applicable to normalization of at least the damaged ATP/ADP ratio in irradiated cells and tissues due to marked capabilities to stimulate the O₂-independent ATP synthesis due to over activation of the ADP- substrate phos phorylation by ²⁵Mg²⁺ cat ions.

According to another feature of the invention the method can be used for treating people suffering hypoxia in the heart muscles as a result of heart attacks, or for people who run the risk of having heart attacks

Preferably, the medicament provides a basic salt of an ion carrier with ²⁵_{M}g²⁺. E.g., the medicament comprises a compound having a porphyrin group.

Preferred compounds having a porphyrin group are porphylleren compound according to formula (i) 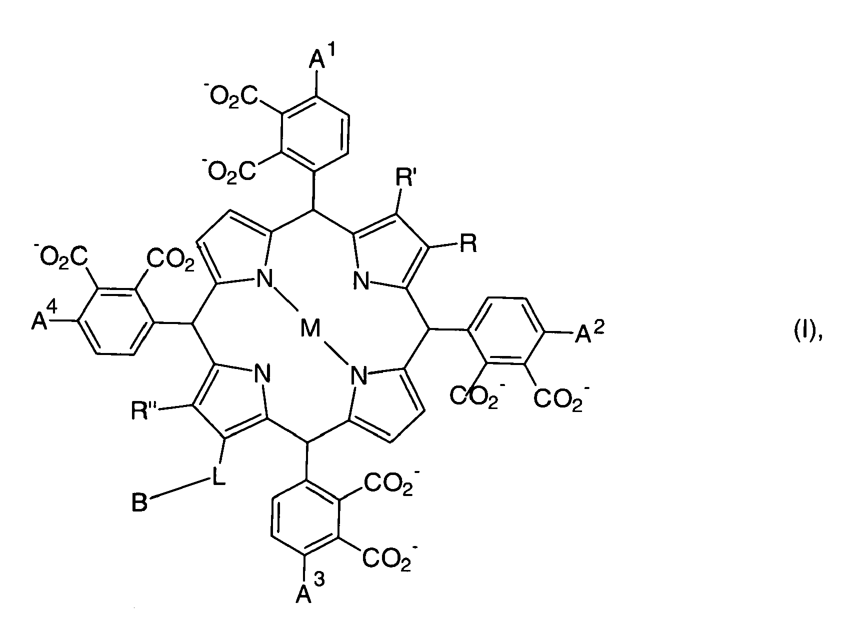 wherein the residues A¹, A², A³, and A⁴ independently are a group containing 1 to 40 carbon atoms and at least one carboxylic group, the residues R, R' and R" independently are hydrogen or a group containing 1 to 80 carbon atoms, M is 2 H or an element capable of complexing with pyrrole nitrogen atom and L is a linking group and B is a fullerene residue.

Preferably, the linking group L is a bond or a group containing 1 to 40 carbon atoms, more preferably 1 to 10 carbon atoms. According to a preferred aspect of the present invention, the linking group L is derived from a butadiene-yl group.

The residue B is a fullerene residue, preferably a fullerene residue having 60 to 80 carbon atoms, more preferably a C₆₀-fullerene.

In Formula (I) M is 2 H or an element capable of complexing with pyrrole nitrogen atom. These elements are well known in the art and include transition metals, e.g. iron.

The residues R, R' and R" independently are hydrogen or a group containing 1 to 80 carbon atoms, preferably 1 to 10 and more preferably 1 to 5 carbon atoms. According to a special embodiment of the present invention, the residues R, R' and R" are hydrogen.

Preferably, the residues A¹, A², A³, A⁴ independently are a group according to formula (II) and/or (III) 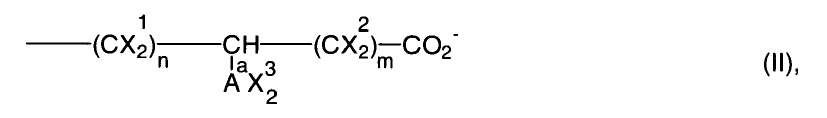 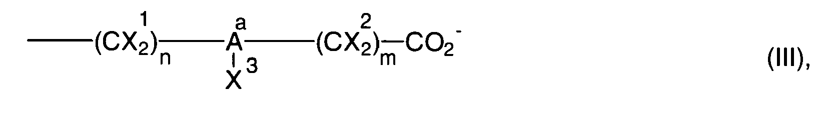 wherein n and m independently are a integer in the range of 0 to 10, A^{a} is nitrogen (N), phosphorus (P), arsenic (As) or a group containing 1 to 40 carbon atoms, the residues X¹, X² and X³ independently are hydrogen, halogen or a group containing 1 to 40 carbon atoms. Preferably, A^{a} is nitrogen (N) or a group containing 1 to 5 carbon atoms and the residues X¹, X² and X³ independently are hydrogen, halogen or a group containing 1 to 3 carbon atoms. According to a preferred aspect of the present invention, the residues A¹, A², A³, A⁴ independently are a group containing 1 to 10 carbon atoms. Preferably, the residues A¹, A², A³, and A⁴ independently are a group containing at least one nitrogen atom.

The expression "group having from 1 to 80 carbon atoms" refers to radicals of organic compounds having from 1 to 80 carbon atoms. The same applies for the expression "group having from 1 to 40 carbon atoms".

These groups include, inter alia, alkyl, cycloalkyl, alkoxy, cycloalkoxy, cycloalkylthio, alkenyl, alkanoyl, alkoxycarbonyl groups and also heteroaliphatic, aromatic and heteroaromatic groups. The groups mentioned can be branched or unbranched.

According to the invention, aromatic groups are radicals of monocyclic or polycyclic aromatic compounds which preferably have from 6 to 20, in particular from 6 to 12, carbon atoms. Heteroaromatic groups are aryl radicals in which at least one CH group has been replaced by N and/or at least two adjacent CH groups have been replaced by S, NH or O, with heteroaromatic groups having from 3 to 19 carbon atoms. Aromatic or heteroaromatic groups which are preferred for the purposes of the invention are derived from benzene, naphthalene, biphenyl, diphenyl ether, diphenylmethane, diphenyldimethylmethane, bisphenone, diphenyl sulfone, thiophene, furan, pyrrole, thiazole, oxazole, imidazole, isothiazole, isoxazole, pyrazole, 1,3,4-oxadiazole, 2,5-diphenyl-1,3,4-oxadiazole, 1,3,4-thiadiazole, 1,3,4-triazole, 2,5-diphenyl-1,3,4-triazole, 1,2,5-triphenyl-1,3,4-triazole, 1,2,4-oxadiazole, 1,2,4-thiadiazole, 1,2,4-triazole, 1,2,3-triazole, 1,2,3,4-tetrazole, benzo[b]thiophene, benzo[b]furan, indole, benzo[c]thiophene, benzo[c]furan, isoindole, benzoxazole, benzothiazole, benzimidazole, benzisoxazole, benzisothiazole, benzopyrazole, benzothiadiazole, benzotriazole, dibenzofuran, dibenzothiophene, carbazole, pyridine, bipyridine, pyrazine, pyrazole, pyrimidine, pyridazine, 1,3,5-triazine, 1,2,4-triazine, 1,2,4,5-triazine, tetrazine, quinoline, isoquinoline, quinoxaline, quinazoline, cinnoline, 1,8-naphthyridine, 1,5-naphthyridine, 1,6-naphthyridine, 1,7-naphthyridine, phthalazine, pyridopyrimidine, purine, pteridine or quinolizine, 4H-quinolizine, diphenyl ether, anthracene, benzopyrrole, benzoxathiadiazole, benzoxadiazole, benzopyridine, benzopyrazine, benzopyrazidine, benzopyrimidine, benzotriazine, indolizine, pyridopyridine, imidazopyrimidine, pyrazinopyrimidine, carbazole, acridine, phenazine, benzoquinoline, phenoxazine, phenothiazine, acridizine, benzopteridine, phenanthroline and phenanthrene, which may also be substituted.

Preferred alkyl groups include the methyl, ethyl, propyl, isopropyl, 1-butyl, 2-butyl, 2-methylpropyl, tert-butyl group, the pentyl, 2-methylbutyl, 1,1-dimethylpropyl, hexyl, heptyl, octyl, 1,1,3,3-tetramethylbutyl, nonyl, 1-decyl, 2-decyl, undecyl, dodecyl, pentadecyl and eicosyl group.

Preferred cycloalkyl groups include the cyclopropyl, cyclobutyl, cyclopentyl, cyclohexyl, cycloheptyl and cyclooctyl group, which may be substituted by branched or unbranched alkyl groups.

Preferred alkenyl groups include the vinyl, allyl, 2-methyl-2-propenyl, 2-butenyl, 2-pentenyl, 2-decenyl and 2-eicosenyl groups.

Preferred alkynyl groups include the ethynyl, propargyl, 2-methyl-2-propynyl, 2-butynyl, 2-pentynyl and 2-decynyl groups.

Preferred alkanoyl groups include the formyl, acetyl, propionyl, 2-methylpropionyl, butyryl, valeroyl, pivaloyl, hexanoyl, decanoyl and dodecanoyl groups.

Preferred alkoxycarbonyl groups include the methoxycarbonyl, ethoxycarbonyl, propoxycarbonyl, butoxycarbonyl, tert-butoxycarbonyl group, hexyloxycarbonyl, 2-methylhexyloxycarbonyl, decyloxycarbonyl or dodecyloxycarbonyl group.

Preferred alkoxy groups include alkoxy groups whose hydrocarbon radical is one of the abovementioned preferred alkyl groups.

Preferred cycloalkoxy groups include cycloalkoxy groups whose hydrocarbon radical is one of the abovementioned preferred cycloalkyl groups.

Preferred heteroaliphatic groups include the abovementioned preferred cycloalkyl radicals in which at least one carbon unit has been replaced by O, S or an NR^{a} group and R^{a} is hydrogen, an alkyl group having from 1 to 6 carbon atoms, an alkoxy group having from 1 to 6 carbon atoms or an aryl group.

Very particular preference is given according to the invention to branched or unbranched alkyl or alkoxy groups having from 1 to 20 carbon atoms, preferably from 1 to 12, advantageously from 1 to 16 and in particular from 1 to 4, carbon atoms and cycloalkyl or cycloalkyloxy groups having from 3 to 20 carbon atoms, preferably 5 or 6 carbon atoms.

Although one or more hydrogen atoms in the abovementioned radicals can be replaced by halogen atoms, preferably chlorine or fluorine, thiol or hydroxy groups or groups of the general formulae NR^{a} R^{b} and N⁺R^{a}R^{b}R^{c}, where the radicals R^{a}, R^{b} and R^{c} are each, independently of one another, hydrogen, an alkyl group having from 1 to 6 carbon atoms, an alkoxy group having from 1 to 6 carbon atoms or an aryl group, unsubstituted radicals have been found to be particularly advantageous.

The formula (I) discloses the compound as an anion. The anionic charge of compound can be balanced by any cation, e.g. protons, ammonium ions and metal ions. Surprisingly, the cation exchange properties (cationite properties) of the compound can be used in order to achieve superior properties as mentioned above. According to one of the aspects of the present invention the compound can be used either in its free form, in the form of a homogenous or heterogeneous complex of one or some organic or in organic cations of different charges. The term homogenous as expressed here, refers to the case of presence of identical cations in the complex form of the compound, while according to our definition the term heterogeneous is used for the case of presence of different organic and/or inorganic cations of identical or different charges in its structure. Preferably, the compound comprises ²⁵Mg²⁺ to balance the charge of the structure as disclosed in formula (I).

Preferably, the medicament comprises a porphyrin-adducted fullerene having improved properties. As used herein, the term "porphylleren compound" means a porphyrin-adducted fullerene. According to a preferred embodiment of the present invention nano-cationite particles of porphylleren compounds are provided. Preferably, the compounds of the present invention are nano particles having a particle size in the range of 0.5 to 20, preferably 1.0 to 5 and more preferably 1.5 - 2.5 nm. Porphylleren-MC16 (PMC16) and their derivatives are preferred examples of the compounds provided by the present invention. Preferred porphylleren compounds simultaneously possess both high degrees of water solubility (460 mg/ml in the case of PMC16) and the ability to be dispersed and retained in lipid layer of biological membranes, due to their chemical structures.

According to a special aspect of the present invention, the medicament comprises fullerene (C₆₀)-2-(butadiene-1-yl)-tetra(o-y-aminobutyryl-o-phtalyl)metaloporphyrin, especially fullerene (C₆₀)-2-(butadiene-1-yl)-tetra(o-y-aminobutyryl-o-phtalyl)ferroporphyrin (PMC16).

Preferably, the medicament comprises a complex of the porphylleren compound with [²⁵Mg²⁺]. The complex molar ratio of the porphylleren compound to ²⁵Mg²⁺ can be in the range of 1:1 to 1:4.

The compounds of the present invention preferably have a low toxicity. Preferably, the toxicity of the compound (DL₅₀ is 1000 mg or less, more preferably the toxicity of the compound DL₅₀ is 2000 mg/kg or less. The DL₅₀ can be measured by using rats (i.v., known methods).

Preferably, the compound according to the present invention is soluble in water. According to a preferred aspect, the solubility is at least 300 mg/ml, more preferably at least 450 mg/ml at pH 7.4.

According to a special aspect of the invention, the compound is membranotropic. Preferably, the claimed compounds may have high cationite capacities (320-370 meqv/mmol (in the case of PMC16)), which make them suitable to be used in cationic complexes.

Surprisingly, the selective delivery of ²⁵Mg²⁺ increases the efficiency of the treatment of the hypoxia suffering muscles and decreases the required dose of ²⁵Mg²⁺ required for the treatment.

On the other hand, the remaining of the drug in the cell, due to its structural and/or chemical properties, and the subsequent smart release of the isotope in the case of hypoxia, will give the mechanism other advantages like being able to be used as a precaution for probable hypoxia attacks.

The application of target-selective ion carriers preferably possessing both high degrees of water solubility and the ability to be dispersed and retained in lipid layer of biological membranes in the form of basic salts of the ion carriers with ²⁵Mg²⁺ is suggested as a preferred mechanism for the delivery of the desired isotope to cells, and the smart release of the isotope during or (in case the drug is used to prevent the effects of hypoxia) at the onset of hypoxia.

The target selectivity of the ion carrier species is adjusted regarding the specific characteristics of the target tissue, designing the chemical structure of the carrier, and the nature of the metal(s) in the metal porphyrin. Most preferably the application of a specific derivative of the mentioned family namely PMC16 *(fullerene(C₆₀)-2-(butadiene-1-yl)-tetra(o-y-aminobutyryl-o-phtalyl)* metaloporphyrin, in which the metal in the metal porphyrin can be chosen from any desired metal(s), in the periodic table and one specific or a mixture of their isotopes either in the naturally abundant form, or in form(s) enriched with one specific isotope, or a combination thereof, is provided.

In the case of the target tissue to be that of heart, the most preferred ion carrier is PMC16 (fullerene(C₆₀)-2-(butadiene-1-yl)-tetra(o-y-aminobutyr yl-o-phtalyl) metaloporphyrin, in which the metal in the metal porphyrin is a magnetic isotope of any desired metal ion, most preferably or Fe(II) and which helps deliver the ²⁵Mg²⁺ complex to any desired target, specially to that of heart.

PMC16 derivatives, in the form of complex with ²⁵Mg²⁺, can accumulate in the target tissue or can be designed to do so, for a certain period of time, decreasing the risks caused by hypoxia and smartly releasing the isotope in the case of a hypoxia.

PMC16, in the form of complex with ²⁵Mg²⁺ can accumulate in the target tissue, especially that of heart, for a certain period, decreasing the risks caused by the unexpected hypoxia attacks.

The release of ²⁵Mg²⁺ ion in the target happens through a smart mechanism, according to which the isotope is released only in the case of hypoxia and the consequent acidosis of the cell, which leads to the hydrolysis of the drug and hence releases of the isotope.

Preferably, a porphyrinic fullerene based 1.8 - 2.0 nm magnesium-carrying particles can be used in order to achieve the objects of the present invention, that is: Porphylleren-MC16, in brief PMC16, which is *a fullerene(C₆₀)-2-(butadiene-1-yl)-tetra(o-y-aminobutyryl-o-phtalyl)ferroporphyrin.*

Preferred compounds being useful in order to achieve the objects of the present invention are amphiphilic, membranotropic, slowly metabolizing and low toxic nanocationite to release magnesium in response to the tissue acidosis only, i.e. not sooner than the hypoxia induced pH acidic shift is in fact occurs in a suffering cell. This "smart behavior" of the particles proposed allowed to use them as an appropriate tool in prevention/correction of various tissue hypoxia-related ATP-depleting syndromes that harms myocardium in Mammals.

In order to improve the targeted drug delivery purposes, some steps can be taken to apply a binding protein receptor for the compound being useful to deliver the ²⁵Mg²⁺, e.g. a PMC16-binding protein receptor for the drug targeting; a receptor mentioned is a specific compound of the heart muscle mitochondria membranes (presumably homologous to the myoglolin B-chain), which provides them an ability to retain the drug molecule for not less than a week after a single i.v. Injection.

The drug may also prevent the energy losses in cells and tissues in case of the prior-to-hypoxia administration. So long-term courses of the drug may serve to simultaneous treatment and prevention of hypoxias in patience with a chronic heart failure and related pathologies as well as it might be efficient to prepare people for any anticipated (forthcomings) physiological situation, which may lead to hypoxia (e. g. sports, heavy physical work, stress).

Because the biological membrane functioning requires (ATP, GTP hydrolysis related energy supply), and as long as the cell life span depends on membrane functioning, the proposed drug will provide an additional "guarantee" for a non-stop (regardless on the oxygen accessibility) energy production mechanism. In the harmful energy losses in the cell in the case of hypoxia and hence prolong the life span of a cell.

Because the excess of bio-oxidation activity in a cell (including all known peroxide- forming reactions) harms the cells membranes, depriving them of being involved properly into pathways, the product may help minimize the consignment energy losses due to the over activation of ATP production in ²⁵Mg-²⁺CK reaction.

Increase in lipid peroxidation rate, may harm not only biological membranes the membrane- associated ATP-synthase and NAD(FAD), Dehydrogenates cascade as well, which in turn create a condition for a sharp ATP production. This might be compensated by activation of the CK- dissected ATP synthesis, which is supposed to be activated by THE presence of ²⁵Mg²⁺.

Both tough-UV and *f*-ionizing radiations are known to induce a truly chaos-leading devastating impact towards the eukaryotic cell energy megalopolis the delivery of ²⁵Mg-²⁺ might be also applicable to normalization of at least the damaged ATP/ADP ratio in irradiated cells and tissues due to marked capabilities to stimulate the O₂-independent ATP synthesis due to over activation of the ADP- substrate phosphorylation by ²⁵Mg²⁺ cations.

As an application of the present drug it can be used for treating people suffering hypoxia in the heart muscles as a result of heart attacks, or for people who run the risk of having heart attacks if the drug can be selectively delivered to the heart tissue.

The synthesis of the preferred porphylleren compound can be carried out according to any suitable procedures. Preferably, the method includes a light induced reaction of the reactants under other desired and optimized reaction conditions and parameters mentioned below.

According to another embodiment of the present invention, any proper solvent or reaction medium can be used in the overall synthesis procedure. However, due to the high solubility of preferred compounds according to the present invention, e.g. PMC 16, some or all parts of the synthesis procedure can be easily and effectively synthesized in aqueous media, which makes the synthesis process environment-friendly and the product clean from any undesired, harmful solvent of any kind.

The general method designed by the inventors is as follows, but any variations in the method, or any other methods, which do not change the nature of the reaction, are also claimed by the inventors.

According to a preferred method that is suggested by the inventors, the molecule can be synthesized in an ultrasonic microwave oven at a temperature between 50 to 150° C, preferably at 90-120 °C. The reaction is performed under electromagnetic wavelengths between 1000-3000 nm, preferably between 1,600-2,200 nm wavelengths with frequencies in the range of 10-100 KHz, more preferably between 40-60 KHz. The preferred reaction time is between 0.5-10 hrs, preferably between 1-5 hrs, and most preferably 2.5 hrs. The extensive studies of the inventors show that, the presence of catalytic amounts of any proper catalyst, either in the form of a metal powder, metal complex, or any other organic or inorganic compound may be useful for the proper execution of the synthesis procedure. These studies revealed that presence of metallic solid, powder, granular or nano-particles of one or a combination of Pt, Au, Mn, W, Hg, Cu, Pb, or as mentioned any other catalytic metal, preferably the presence of Pt powder catalyst suspending permanently in pyridine or any other proper organic solvent can be extremely useful for the proceeding of the reaction. Research performed by the inventors shows that presence of 5-100 p, preferably 10-70 µ, and most preferably of 20-40 p metallic granular Pt powder catalyst suspending permanently in 40-80% pyridine, (2.0-.35 mg/cm³) can lead to the best results in terms of yield and purity of the product and reaction parameters.

According to the proposed synthesis method an original modification of the classical Prato reaction was employed according to which a simple Diels-Alder adduct of fullerene C₆₀ or any other proper fullerene family member, with 2-(butadiene-1-yl)teteraphenylporphyrin or any other proper reactant or the mixture of them (depending on the desired derivative) was obtained with a yield of about 97-100%.

The reaction of these diene(s) with C₆₀-fullerene or other desired fullerene family members was perfectly completed in two hrs, whereas cycloadditions with N-phenylmaleimide (or naphtoquinone) usually may require 46-48 hrs.

To generate the above named adduct or any of its proper derivatives, the pyrrolidinefullerene-forming 1,3-dipolar cycloaddition of corresponding azomethynylides was first performed. The latter compounds, in turn, were generated by a direct decarboxylation of immonium salts resulting due to a condensation between α-aminoacid (L-prolyne) or its derivatives and the classical Prato's pyrrolidine-porphyrinic aldehydes.

Being covalently bound to the fullerene family acceptor chromophore, the porphyrin domain played a donor-compound role. The PMC16 porphyrin-fullerene diade, or its desired derivative, was completed apart in a way of the porphyrin domain reattachment to the Prato reaction-generated fullerene derivative, 4-(*N*-methyl-3,4-fullerene-pyrrolydine) phtalenonitryl or any of its derivatives. This step was carried out at boiling conditions in an inert gas atmosphere, e.g. Ar or other noble gasaerating, by using pure toluene or any other proper organic or inorganic solvent for 1-6 hrs, preferably 4 hrs.

This step can be followed by a following 2-10 hrs, preferably 6 hrs long incubation of the product with proper concentrations, preferably 1.0-3.0 M, most preferably with 2.0 M of any soluble metal salt solution.

The metal solution is chosen according to the needs or desires and can be chosen from any of the metallic or non-metallic actions, preferably, from soluble solutions of transition or heavy metal salts (with different anions and crystal waters) most preferably that of FeCl₂/1.0 M dimethylformamide in boiling O-dichlorobenzene or any other proper solvent.

The "loading" of the porphylleren compound with ²⁵Mg²⁺ cations (preparation of PMC16[²⁵Mg²⁺]n, where n is the number of ²⁵Mg²⁺ and can be any number with respect to the structure of the carrier, and is preferred to be 4 in the case of PMC16), can be done by using solutions comprising ²⁵Mg²⁺. A typical method, based on the investigations of the inventors, PMC16 "loading" is mentioned in example 1. This is just a typical example and any of its variations are also claimed by the inventors and can be used for the same purpose.

The invention is illustrated in more detail below by a preparation example, without intending to limit the invention to these examples.

### Example 1: Preparation of PMC16[²⁵Mg²⁺]₄

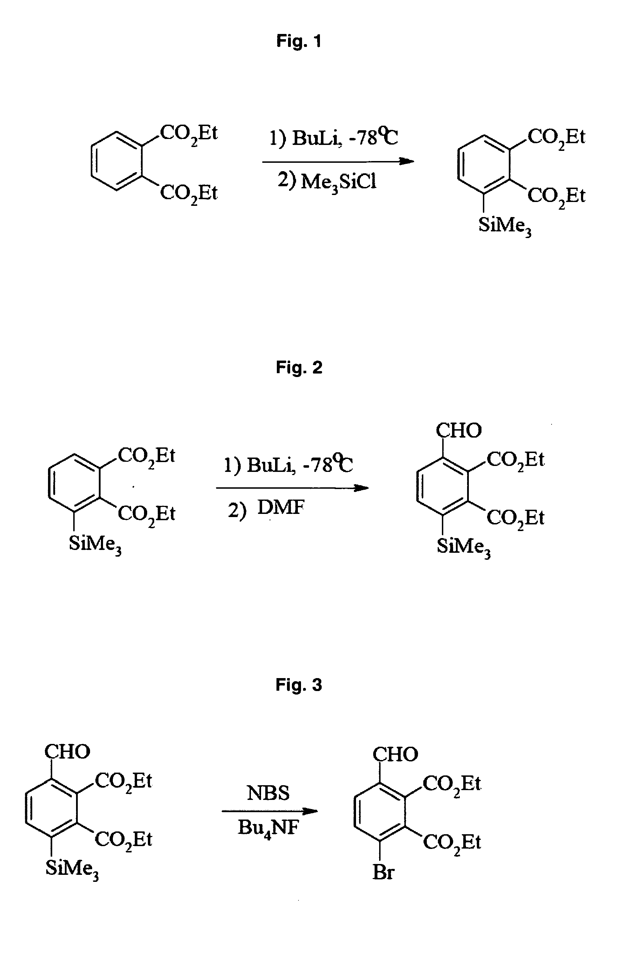

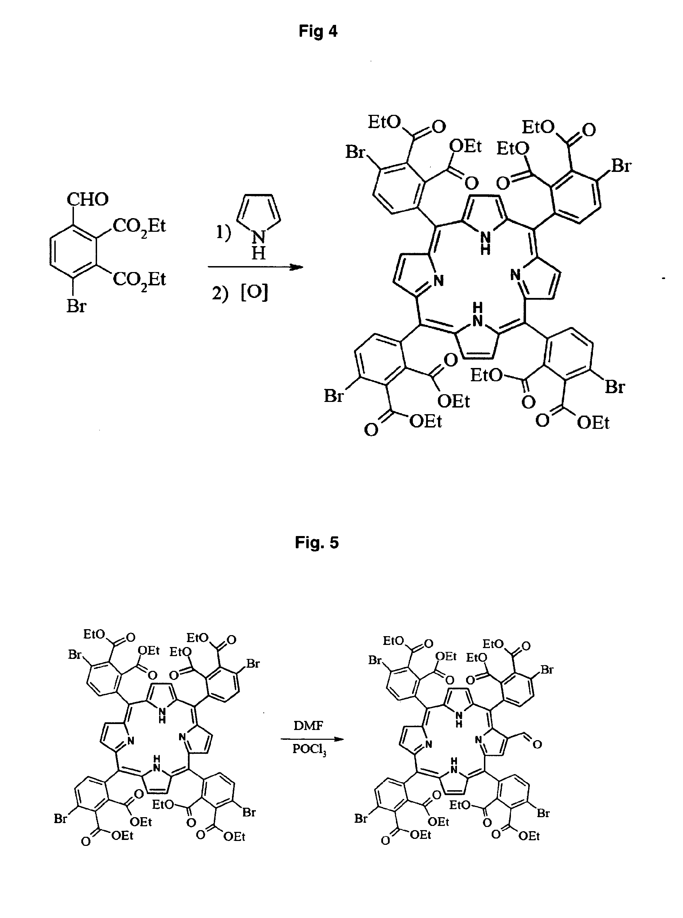

The compound fullerene (C₆₀)-2-(butadiene-1-yl)-tetra(o-y-aminobutyryl-o-phtalyl)ferroporphyrin (Porphylleren-MC16 or PMC 16) can be prepared by the following method which method is exemplified in the scheme as mentioned in figures 1 to 9.

First a diethyl phthalate is to be silicomethylated by the trimethylsilicochloride in the presence of the lithium butyrate catalyst with a formation of the diethyl-3-(trimethylsilyl)phthalate (product i) in Fig. 1. The latter one is then transforms into the diethyl-3-formyl-6-(trimethylsilyl)phthalate (Product II) by oxidation of the product I in the presence of the same mentioned catalyst and the dimethylazomethanol (see Fig. 2). The product II is then momobromated by Nbromosuccinimide in the presence of the butyrylazofluoride so the diethyl-6-bromo-3-formylphthalate (Product III) formed as a result (see Fig. 3). Next, the Product III is to be condensated with pyrrol in the 2,3-dichloro-5,6-dicyano-1 m4-benzoquinone (DDQ) presence which leads to formation of the Product IV, the first obtained porphyrin structure (see Fig. 4). Then the Product IV transforms into the Product V (see Fig. 5) in the presence of dimethylazomethanol and POCl₃.

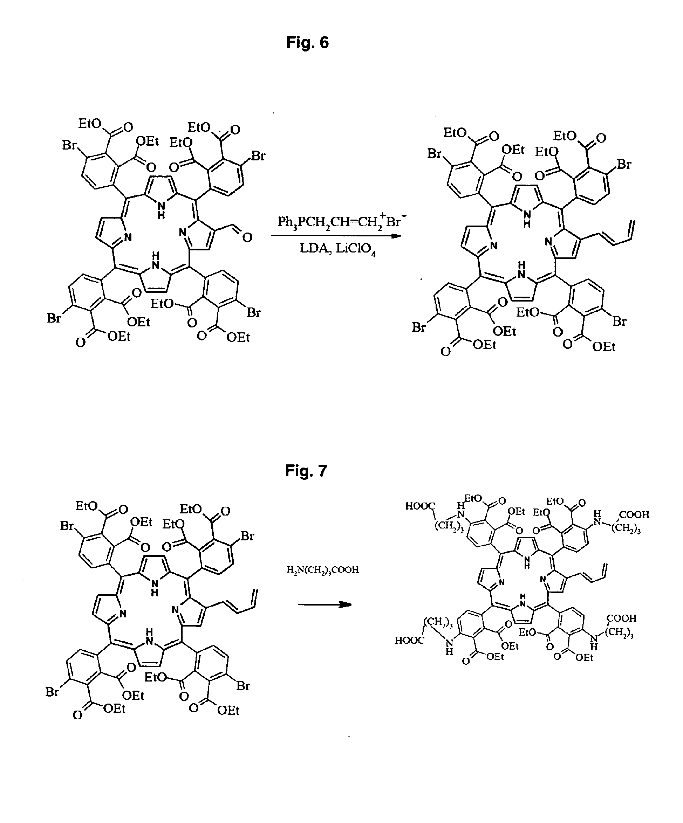

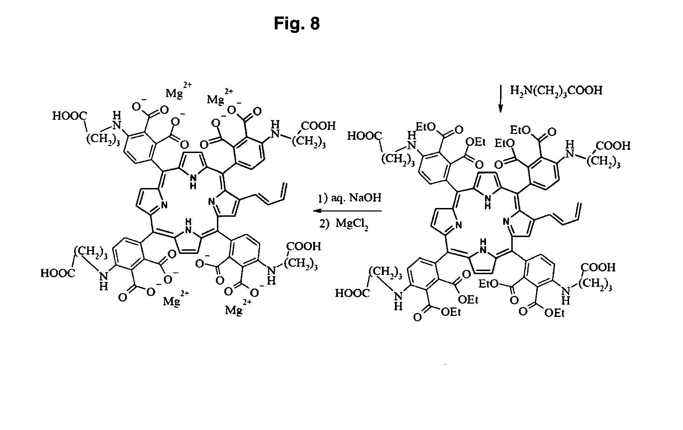

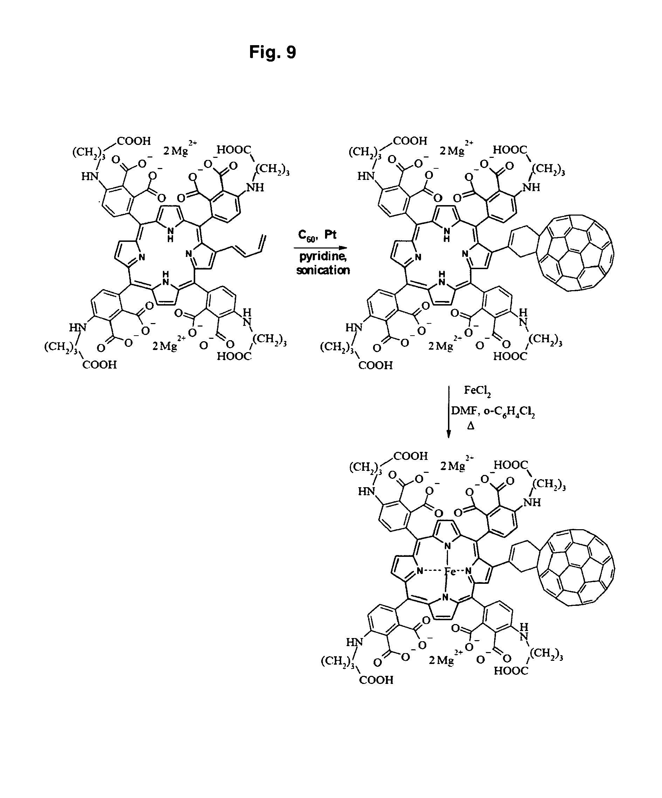

Then the Product V transforms into the Product VI by the one-step combined treatment with lithium disopropylamide (LDA) and the lithium perchlorate to form Product VI (see Fig. 6). Dimethylaminoacetic acid is to induce the transformation of the Product VI into the Product VII like indicated in the scheme (see Fig. 7). Simple addition of NaOH and MgCl2 transforms Product VII into the Product VIII which, in turn, transformes then into the fullerene porphyrin adduct by the treatment with C60-fullerene, Pt catalyst (powder suspension), here a simple pyridine sonication is involved. Eventually, the final PMC16 nanostructure is to be formed from the Product IX by incubation (combined simultaneous treatment) with FeCl2, butyryldichloride and dimethylazoethanol (see Fig. 8 and 9).

PMC16 "loading" with ²⁵Mg²⁺ cations(saturation,PMC16[Mg²⁺]4) is done according to a following procedure. Up to 600-800 mg of PMC16 is dissolved in 1.0 mL of mixture of pyridine: CS₂: chloroform or any other suitable solvents (with a ration of 1:2:7) at the room temperature. Then 4 volumes of 2.5M solution of pure ²⁵MgCl₂ (98.6% isotopic purity) in 0.2M NAOH is added to the PMC16-containing organic mixture. After 30 min of extensive shaking (at 22-25°-C), the water-soluble phase is separated by centrifugation at 15000 r.p.m 20 min, 22 °C, carefully collected and then lyophilized (or evaporated). Following massspectroscopy and atomic absorption spectroscopy data are used to control the Mg/Fe ratio in the produced PMC16 complex.

The remaining organic phase was repeatedly (2-3 times) subjected to Mg²⁺ alkaline water extraction as described above. The organic phasecontaining ²⁵MgCl₂ traces are then re-utilized. Using the fast rotor evaporating and a consequent dissolving in 10mM sodium-phosphate (pH=9.60) buffer containing 15Mm EDTA. The very same way to concentrate the resulted PMC16[Mg²⁺]₄ is applied to the first alkaline-water extraction run.

### Example 2: In vivo tests on basic pharmacokinetics and some biotransformation patterns of the drug on rats

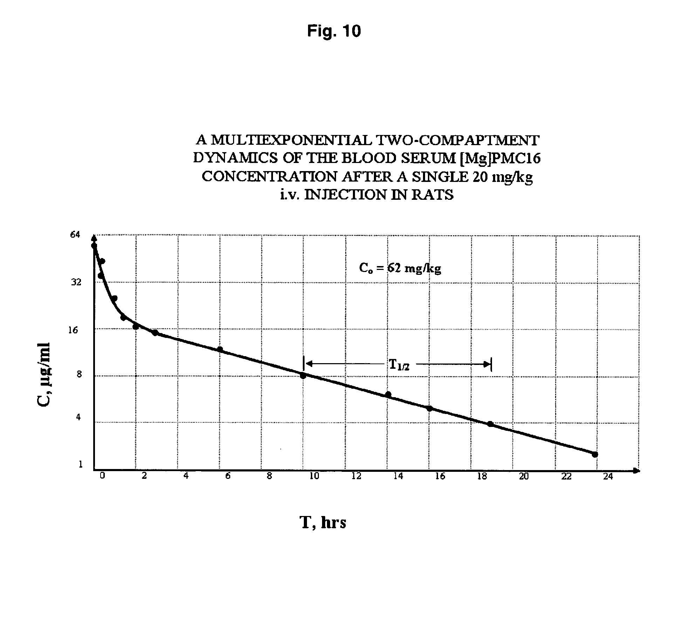

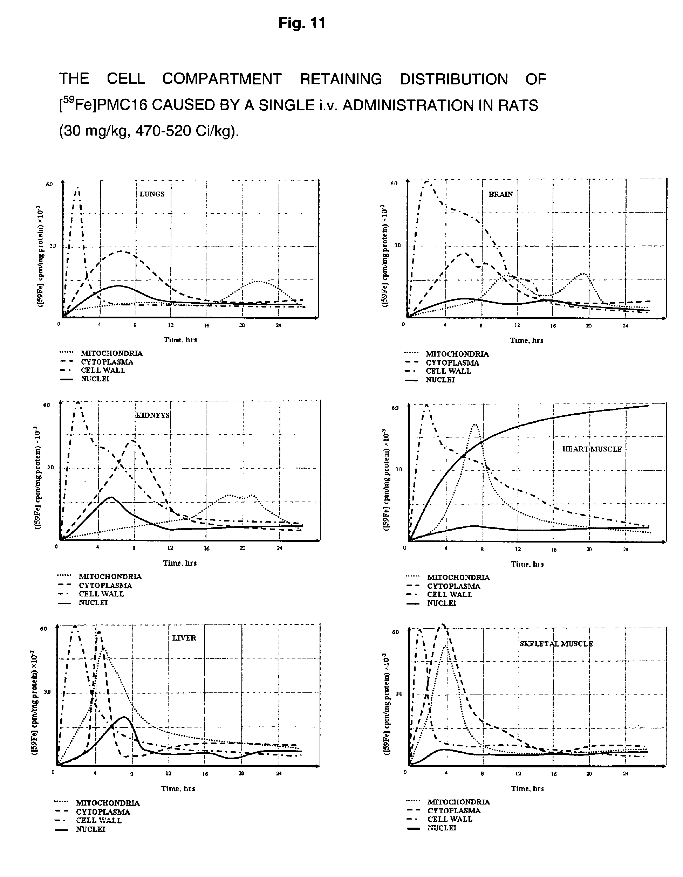

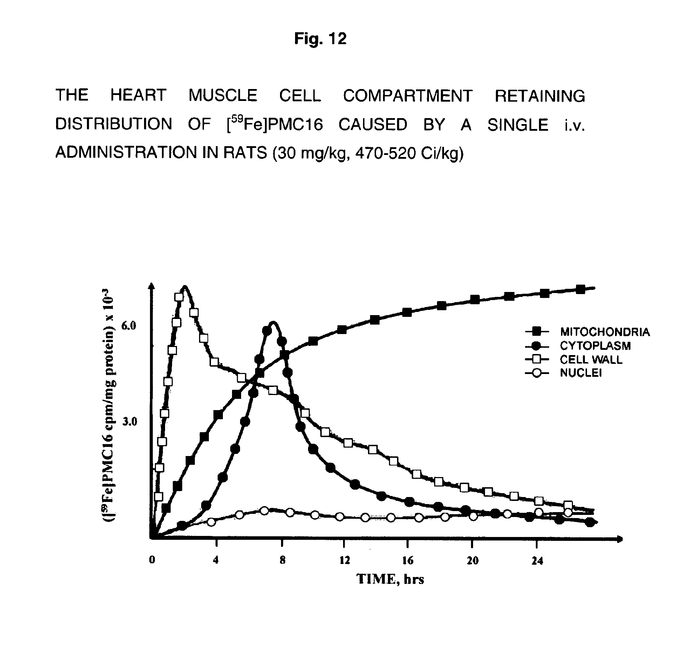

Considering an essential pharmacological potential of PMC16[Mg²⁺]₄, its basic pharmacokinetics along with some biotransformation patterns were estimated by in vivo tests carried out with rats (Fig.10-13, Table 1).

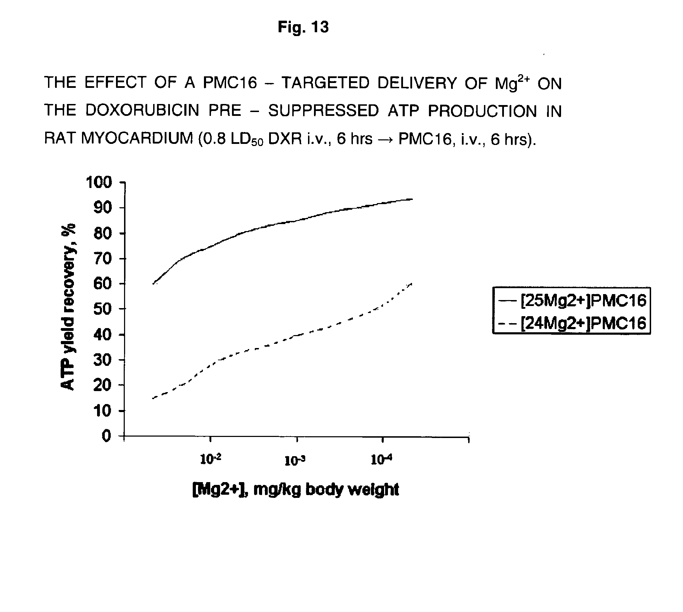

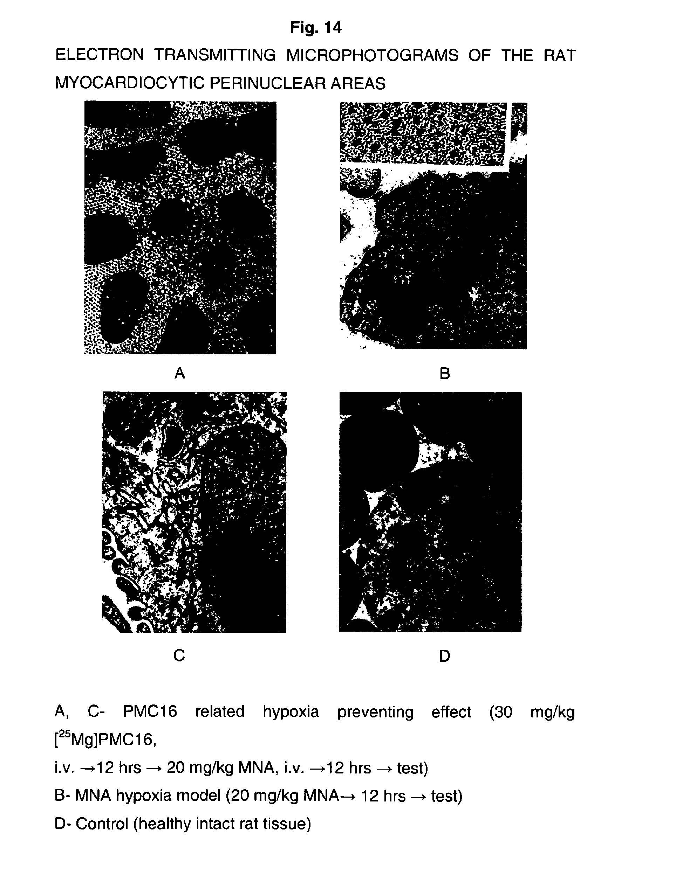

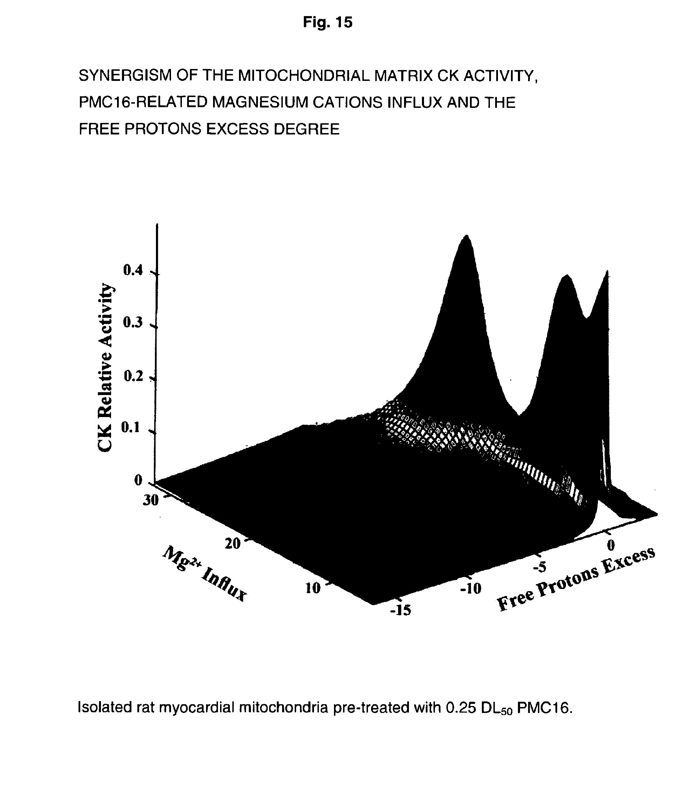

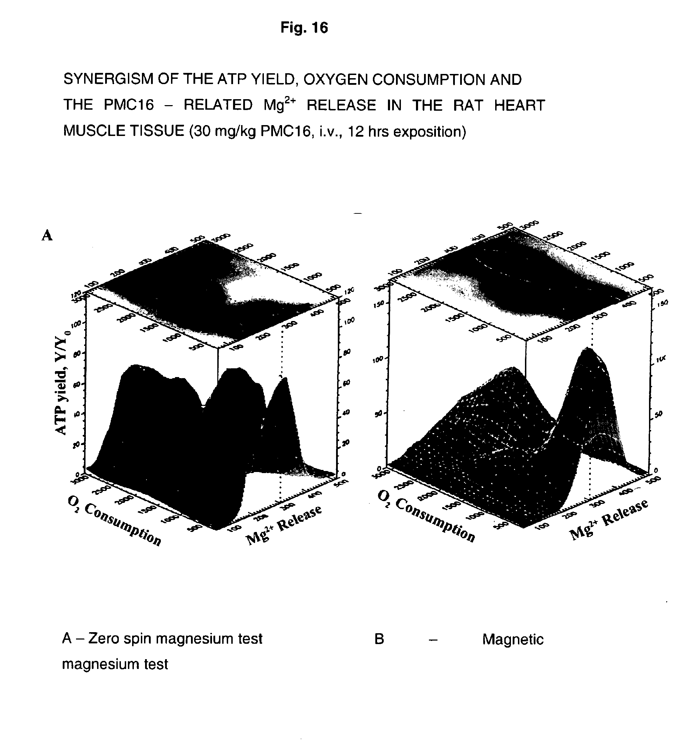

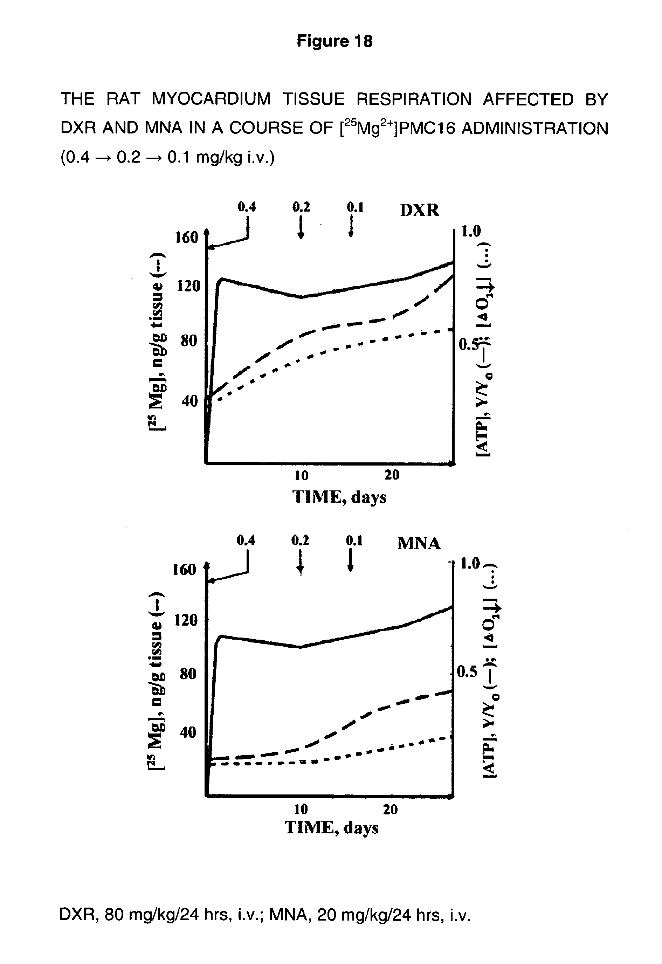

Besides, a biochemical/pharmacological study on PMC16[Mg²⁺]₄ effects in rat myocardium affected by the drug (Doxorubicin [DXR], 1-methylnicotineamide [MNA]) induced hypoxia were conducted to prove both drug efficiency and safeness with respect to cationite properties of the new nano medicament (Fig. 13,18). Same tests were also performed in inhalation hypoxia model specified below (Fig.14-16).

The following in vivo experimental hypoxia models were employed:
(1) Medicinal hypoxia syndrome A (1-methylnicotineamide (MNA), 10-20 mg/kg i.v. once per 24 hrs);
(2) Medicinal hypoxia syndrome B (DXR 60-80 mg/kg i.v. once per 24 hrs);
(3) The oxygen depleted inhalation model (10-20 day long exposition of rats to the artificial gas mixture to breath with, 12-16% oxygen / 84-88% nitrogen, v/v).

Male adult Wistar Albino Glaxo rats (180-220 gms) were used, 3-4 animals per each experimental point.

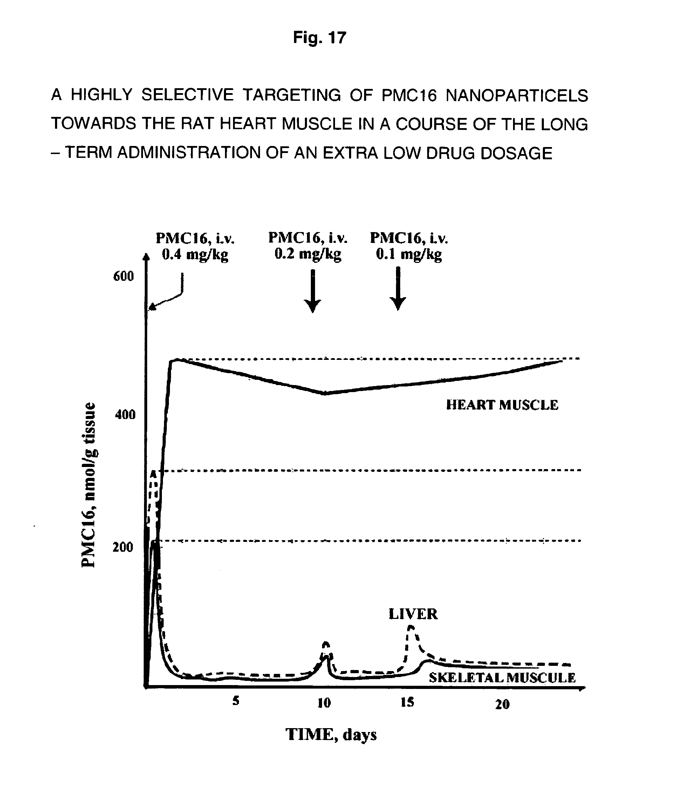

As seen from results of all tests conducted (Table 1, Fig. 10-18) the proposed product (PMC16) is indeed efficient for protecting the heart muscle tissue from the damaging impact promoted by hypoxia of any sort, even when the extra low doses of the drug administrated (Fig. 17).

Particularly, due to the PMC16-specific receptor located in the myocardiocyte mitochondria membranes, a selective targeted delivery of the drug might be managed simply by a long term multiple administration of the drug extra low doses. Being totally safe, this course provides a marked prophylactic and therapeutic effect in medicinal hypoxic cardiotoxicity cases (DXR, MNA)- Fig. 13, 16,18.

**Table 1:**

| **THE [Mg]PMC16 PHARMACOKINETICS IN RAT** | |
|---|---|
| **Single 20mg/kg i.v. injection (M ± SEM, n = 6)** | |
| **24 hrs monitoring presented** | |
| T_{½} = 9. 0 hrs | C₀ = 62 µg/ml |
| Tₘₐₓ = 2.5 hrs | Cₘₐₓ = 260 ± 83 ng/ml |
| Cl = 32 ±4 ml/min/kg | V_{P}= 16. 2 ml/kg |
| *k* = 0. 685 | V_{c} = 12.4 ml |
| | V₁= 0.08 ml |
| | |
| Renal excretion: | 28 ±4.3% |
| Hepatic excretion: | 16 ±4.0% |
| (metabolization) | |
| Plasma proteins binding: | 1. 2± 0.3% |
| BLOOD CELLS UPTAKE | |
| Lymphocytes: | 28.6 ± 5.5% |
| Erythrocytes: | 8.0 ± 3.2% |
| TISSUE SPECIFIC ACCUMULATION | |
| Myocardium: | 18.4 ±3.40% |
| Brain: | 0.6 ±0.02% |
| URINE ELIMINATING PMC16 METABOLITES | (258 ± 4. 0 µg/ml) |
| Alanyl-depleted derivatives: | 56.4 ± 8.7% |
| Deacetylated derivatives: | 27.0 ± 6.1% |
| Cyclohexyl -C60: | 16.2 ± 3.3% |
| PMC16 urine content: | 462 ± 11 µg/ml |
| C60 urine content: | 2.9 ± 0.1 µg/ml |

## Claims

1. A use of the magnetic magnesium isotope ²⁵Mg²⁺ for the manufacture of a medicament for treating hypoxia.

2. The use according to claim 1 wherein the medicament provides a targeted and selective delivery of the ²⁵Mg²⁺to any muscle.

3. The use according to claim 1 or 2 wherein the medicament corrects the energy metabolism disorders in myocardiocytes.

4. The use according to at least one of the preceding claims wherein the medicament increases the efficiency of the treatment of the hypoxia suffering muscles and decreases the required dose of ²⁵Mg²⁺ required for the treatment.

5. The use according to at least one of the preceding claims wherein the medicament comprises a target-selective ion carrier.

6. The use according to claim 5 wherein the ion carrier possesses a high degree of water solubility and the ability to be dispersed and retained in lipid layer of biological membranes.

7. The use according to at least one of the preceding claims wherein the medicament provides a selective delivery and smart release of the Mg²⁺ isotope.

8. The use according to claim 7 wherein specific structural, magnetic, and/or chemical properties of the tissue is used for the selective delivery and smart release.

9. The use according to at least one of the preceding claims wherein the medicament provides a basic salt of an ion carrier with ²⁵Mg²⁺.

10. The use according to at least one of the preceding claims wherein the medicament comprises the magnesium isotope ²⁵Mg²⁺ in the form of a complex and the complex is able to accumulate in the target tissue for a certain period of time, and can be smartly released in the target tissue only after hypoxia attacks.

11. The use according to at least one of the preceding claims wherein the release of ²⁵Mg²⁺ ion in the target happens through an intelligent mechanism, according to which the isotope is only released in the case of hypoxia.

12. The use according to at least one of the preceding claims wherein ²⁵Mg²⁺ is only released quantitatively in the target tissue, in the case of the hypoxia and the consequent acidosis and due to the hydrolysis of the drug.

13. The use according to at least one of the preceding claims wherein the medicament prevents the energy losses in cells and tissues in case of the prior-to-hypoxia administration.

14. The use according to at least one of the preceding claims wherein the medicament serves to simultaneous treatment and prevention of hypoxias in patients with a chronic heart failure and related pathologies in long-term courses.

15. The use according to at least one of the preceding claims wherein the medicament provides a non-stop energy production mechanism by which harmful energy losses in the cell in the case of hypoxia can be prevented and hence prolongs the life span of a cell.

16. The use according to at least one of the preceding claims wherein the medicament minimizes the consignment energy losses due to the over activation of ATP production in ²⁵Mg-2⁺CK reaction.

17. The use according to at least one of the preceding claims wherein the medicament creates a condition for a sharp ATP production, which might be compensated by the activation of the CK-dissected ATP synthesis supposed to be activated by the presence of ²⁵Mg²⁺.

18. The use according to at least one of the preceding claims wherein the delivery of ²⁵Mg-²⁺ is applicable to normalization of at least the damaged ATP/ADP ratio in irradiated cells and tissues due to marked capabilities to stimulate the 02-independent ATP synthesis due to over activation of the ADP- substrate phosphorylation by ²⁵Mg²⁺ cations.

19. The use according to at least one of the preceding claims wherein the medicament can be administered to the patients by inhalation, injection or oral application forms.

20. The use according to at least one of the preceding claims wherein the medicament comprises a compound having a porphyrin group.

21. A medicament for treating hypoxia comprising the magnetic magnesium isotope ²⁵Mg²⁺ and a porphylleren compound according to formula (1) 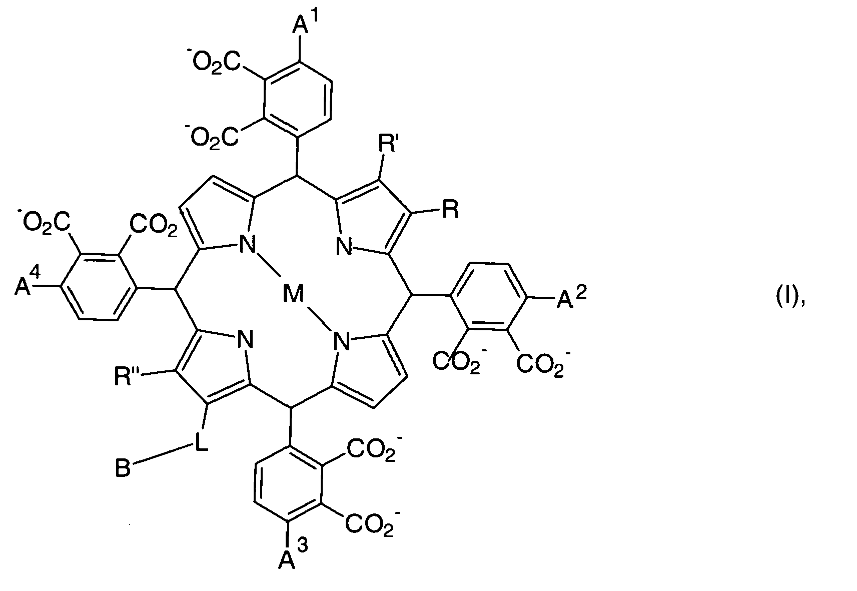 wherein the residues A¹, A², A³, and A⁴ independently are a group containing 1 to 40 carbon atoms and at least one carboxylic group, the residues R, R' and R" independently are hydrogen or a group containing 1 to 80 carbon atoms, M is 2 H or an element capable of complexing with pyrrole nitrogen atom and L is a linking group and B is a fullerene residue.

22. The medicament according to claim 21 wherein the toxicity of the compound DL₅₀ is 2000 mg/kg or less.

23. The medicament according to claim 21 or 22 wherein the solubility is at least 450 mg/ml at pH 7.4.

24. The medicament according to at least one of the preceding claims 21 to 23 wherein the compound is membranotropic.

25. The medicament according to at least one of the preceding claims 21 to 24 wherein the fullerene residue is a C₆₀ fullerene.

26. The medicament according to at least one of the preceding claims 21 to 25 wherein the residues R, R' and R" independently are hydrogen or a group containing 1 to 5 carbon atoms.

27. The medicament according to at least one of the preceding claims 21 to 26 wherein the residues R, R' and R" are hydrogen.

28. The medicament according to at least one of the preceding claims 21 to 27 wherein the residues A¹, A², A³, A⁴ independently are a group according to formula (II) and/or (III) 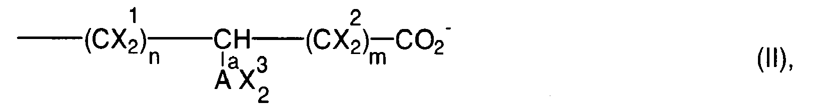 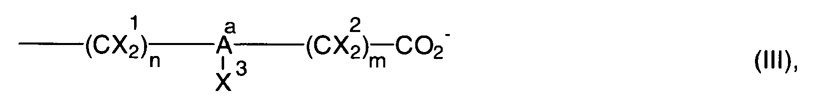 wherein n and m independently are a integer in the range of 0 to 10, A^{a} is nitrogen (N), phosphorus (P), arsenic (As) or a group containing 1 to 40 carbon atoms, the residues X¹, X² and X³ independently are hydrogen, halogen or a group containing 1 to 40 carbon atoms.

29. The medicament according to claim 28 wherein A^{a} is nitrogen (N) or a group containing 1 to 5 carbon atoms and the residues X¹, X² and X³ independently are hydrogen, halogen or a group containing 1 to 3 carbon atoms.

30. The medicament according to at least one of the preceding claims 21 to 29 wherein the residues A¹, A², A³, A⁴ independently are a group containing 1 to 10 carbon atoms.

31. The medicament according to at least one of the preceding claims 21 to 29 wherein the residues A¹, A², A³, and A⁴ independently are a group containing at least one nitrogen atom.

32. The medicament according to at least one of the preceding claims 21 to 31 wherein M is a transition metal.

33. The medicament according to claim 32 wherein wherein M is iron.

34. The medicament according to at least one of the preceding claims 21 to 33 wherein the linking group L comprises 1 to 10 carbon atoms.

35. The medicament according to claim 34 wherein the linking group L is derived from a butadiene-yl group.

36. The medicament according to at least one of the preceding claims 21 to 35 wherein the compound is fullerene (C₆₀)-2-(butadiene-1-yl)-tetra(o-Y-aminobutyryl-o-phtalyl)ferroporphyrin.

37. The medicament according to at least one of the preceding claims 21 to 37 wherein the medicament comprises a complex of the porphylleren compound with [²⁵Mg²⁺].

38. The medicament according to claim 37 wherein the complex molar ratio of the porphylleren compound to ²⁵Mg²⁺ is in the range of 1:1 to 1:4.
